(19) Europäisches Patentamt
European Patent Office
Office européen des brevets

(11) EP 3 233 060 B1

(12) # FASCICULE DE BREVET EUROPEEN

(45) Date de publication et mention de la délivrance du brevet:
**05.02.2020 Bulletin 2020/06**

(21) Numéro de dépôt: **15817481.3**

(22) Date de dépôt: **07.12.2015**

(51) Int Cl.:
***A61K 9/16*** *(2006.01)* ***A61K 9/00*** *(2006.01)*

(86) Numéro de dépôt international:
**PCT/FR2015/053345**

(87) Numéro de publication internationale:
**WO 2016/097525 (23.06.2016 Gazette 2016/25)**

(54) **LIBÉRATION MAÎTRISÉE DE SUBSTANCES ACTIVES**

KONTROLLIERTE FREISETZUNG VON WIRKSTOFFEN

CONTROLLED RELEASE OF ACTIVE SUBSTANCES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.12.2014 FR 1462413**

(43) Date de publication de la demande:
**25.10.2017 Bulletin 2017/43**

(73) Titulaire: **Société d’Exploitation de Produits pour les Industries Chimiques SEPPIC**
**75321 Paris Cedex 7 (FR)**

(72) Inventeurs:
- **DUCCINI, Yves**
**81570 Vielmur/agout (FR)**
- **TROUVE, Gérard**
**81100 Castres (FR)**

(74) Mandataire: **Laigneau, Amandine**
**L’Air Liquide**
**Direction Propriété Intellectuelle**
**75, Quai d’Orsay**
**75321 Paris Cedex 07 (FR)**

(56) Documents cités:
**WO-A1-97/48410**
**WO-A2-2007/018943**
**US-A1- 2003 104 048**
**US-A1- 2004 062 778**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 233 060 B1

## Description

La présente invention concerne la libération de principes actifs pharmaceutiques, alimentaires ou vétérinaires dans le corps de l'homme ou de l'animal suivant une cinétique totalement maîtrisée et en masquant des goûts désagréables par une technologie de perlage (« prilling » en anglais) ou de revêtement par fusion (« hot melt coating » en anglais).

L'industrie pharmaceutique, l'industrie des compléments alimentaires et l'industrie vétérinaire mettent en œuvre des formulations ayant de plus en plus de molécules complexes, au goût souvent désagréable, parfois fragiles, et qui doivent se diffuser dans les organismes suivant une cinétique bien particulière.

En effet pour apporter les avantages nutritionnels ou thérapeutiques visés, les molécules introduites dans des capsules, des gélules, des comprimés voire dans de la nourriture ne doivent pas se dissoudre à la même vitesse et dans le même environnement.

En particulier les molécules sont détruites lorsqu'elles circulent dans l'estomac humain ou celui de l'animal. Son pH très acide et la présence de sucs digestifs et d'enzymes font que peu de molécules parviennent à traverser intactes le passage dans cet organe qui va durer une à deux heures chez l'humain et beaucoup plus longtemps chez le ruminant.

De plus, la prise de médicament ou de complément alimentaire est souvent associée à une ingestion de nourriture (quasi systématique chez l'animal) qui accroit encore les réactions chimiques pouvant dégrader certaines molécules.

A l'inverse la libération rapide d'une partie sinon de toute la quantité de matière active est parfois recherchée de façon à obtenir un effet quasi immédiat. C'est le cas par exemple d'anti-vomitifs, d'antalgiques ou d'anti-inflammatoires non stéroïdiens.

Dans certains cas on veut conférer à la molécule introduite une propriété dite gastro-résistante. Il est alors souhaitable que la molécule active ne se libère qu'après le passage stomacal, c'est le cas de molécules souvent fragiles et agissant sur la digestion comme les anti-oxydants, les anti-bactériens, les souches régénérant la flore intestinale (prébiotiques) et celles facilitant sa croissance (pro biotiques), certains antibiotiques et anti-inflammatoires. Souvent l'homme de l'art veut même contrôler à quel endroit de l'intestin va se libérer la molécule active. L'intestin est très long et le temps de transit peut être important.

Chez les ruminants, on l'estime entre quatre et cinq heures dans l'intestin grêle et entre quarante à soixante heures dans le gros intestin. Régler à quelle vitesse et donc à quel endroit de l'intestin se libèrera la molécule active est très difficile mais très bénéfique pour l'efficacité de la molécule.

Beaucoup de molécules utilisées dans la pharmacie, les compléments alimentaires ou les produits vétérinaires ont en outre un goût désagréable, amer ou astringent. L'homme de l'art voudra cacher ces goûts tout en contrôlant, comme décrit ci-dessus, les cinétiques de diffusion dans l'organisme.

Outre les exemples de molécules médicamenteuses déjà décrits, l'appauvrissement de la nourriture conduit les sociétés de compléments alimentaires et même les sociétés de l'alimentaire proprement dit, à rajouter dans leurs produits des molécules dont l'humain ou l'animal manque. Beaucoup de molécules, lorsqu'elles sont utilisées à l'état pur, ont un goût désagréable ou sont trop fragiles pour résister à l'estomac ou aux procédés de fabrication des aliments (cuisson, pasteurisation, atomisation ...).

On peut citer encore une fois les pro et pré biotiques mais aussi les anti-oxydants, les vitamines, les sels minéraux, certains acides aminés.

De nombreuses solutions d'encapsulation, d'absorption ou de pelliculage ont été développées par les hommes de l'art pour résoudre les problèmes préalablement décrits.

Nulle n'est totalement satisfaisante et surtout nulle ne résout à la fois les problèmes de goût et de libération très précise dans le système digestif.

Parmi ces solutions on peut citer : l'absorption sur des supports poreux tels que les silices, les celluloses, les polyméthacrylates ou polyacrylates.

Les inconvénients majeurs de l'absorption sur support poreux sont :

- la faible quantité d'actif absorbé,
- les granulométries des supports rendant perceptible leur utilisation par le consommateur,
- l'impossibilité d'absorber certains actifs,
- la quasi impossibilité de contrôler la libération qui se fait généralement par « échange » entre le produit absorbé et le milieu qui l'entoure.

Ajoutons que le goût est souvent encore perceptible.

Le pelliculage des matières actives par des solutions de sucres ou de polymères au moyen de techniques appelées « pan coating » est de loin la technique la plus utilisée en industrie pharmaceutique pour masquer le goût et faire notamment des comprimés gastro-résistants.

La technique consistant à enrober des noyaux solides par des pellicules de cire (ou « hot melt coating ») est également connue. On peut par exemple citer les brevets de la société Balchem numéro WO 2004/060074 A1 ou US6835397. La technique décrite dans ces brevets fait appel à des produits auto émulsionnables comme excipients de pelliculage. Par ailleurs il n'est pas possible de régler précisément les libérations d'actifs et la technique nécessite que l'actif soit exposé à une chaleur importante pendant plusieurs minutes, pouvant alors générer sa dégradation. Enfin, l'effet conjoint de masquage de goût n'est pas décrit.

Cette technique ne permet pas de régler la vitesse de libération autrement qu'en ajustant la quantité de cire déposée sur le noyau, ce qui peut devenir problématique si l'on désire une libération sur de longues périodes de temps puisque la vitesse de libération est inversement proportionnelle à la quantité de cire déposée.

Enfin le « prilling » est une technologie qui permet de former des microbilles constituées d'un principe actif dispersé dans une cire. Elle est décrite dans quelques articles et brevets mais généralement, cette technologie est effectuée à l'aide de cires du commerce et ne permet pas de régler la vitesse de dissolution dans l'organisme. Elle sert seulement à un masquage de goût et une libération ralentie est réclamée sans plus de précision.

A titre d'exemple on peut citer le brevet CN 101513392 dans lequel sont listés énormément d'excipients pouvant être utilisés mais ne prétend pas réguler la vitesse de libération.

Le document WO2007018943 divulgue quant à lui une composition pharmaceutique comprenant un substrat solide avec une couche d'un actif comme le progesterone, un mélange d'un tensioactif hydrophile (Solulan C-24 HLB = 14) et de deux composants lipophiles (acide desoxycholique et monoglycerides distillés HLB=4).

C'est pourquoi l'invention a pour objet une composition insoluble dans l'eau, d'aspect solide à une température inférieure ou égale à 20°C, comprenant pour 100% de sa masse :

- X1% massique d'au moins un tensioactif lipophile ayant une valeur HLB, H1, supérieure ou égale à 1 et inférieure à 10 ;
- X2% massique d'au moins un tensioactif hydrophile ayant une valeur HLB, H2, supérieure ou égale à 10 et inférieure ou égale à 20 ;

caractérisée par le fait que son HLB = X1.H1 + X2.H2, X1 et X2 variant de 2 à 60 et caractérisée en ce qu'elle est exempte de polymère acrylique et/ou de succinate acétate, et caractérisée en ce que :

- ledit au moins un tensioactif lipophile est un stéarate de sorbitan,et
- ledit tensioactif hydrophile est choisi parmi les esters de sorbitan éthoxylés, les alcools ou acides éthoxylés, les esters de polyglycérol, les éthers de glucose, les copolymères blocs d'oxydes d'éthylène et de propylène.

Par ailleurs, selon d'autres modes de réalisation, l'invention a aussi pour objet :

- Une composition telle que définie ci-dessus caractérisée en ce que H1 est supérieure ou égale à 3,4 et inférieure ou égale à 6 et H2 est supérieure ou égale 14 et inférieure ou égale à 16.
- Une composition telle que définie ci-dessus caractérisée en ce qu'elle comprend de deux à cinq tensioactifs ayant chacun un HLB, Hi, et une proportion massique Xi tels que la valeur HLB de ladite composition est égale à $\sum$ Xi.Hi, avec i compris entre 2 et 5, xi compris entre 2% et 60% et $\sum$ Xi $\leq$ 100%.
- Une composition telle que définie ci-dessus caractérisée en ce que ledit au moins un tensioactif lipophile est choisi parmi :
    - les esters d'acides gras et de sucres ; les dits acides gras étant choisis parmi les acides stéariques, palmitiques, cetostéariques, arachidiques, béhéniques ; les dits sucres étant par exemple du glucose, du sorbitol, du mannitose, du mannitol, du saccharose, du mannose, du xylitol ou du xylose;
    - les esters d'acides gras et de glycérol, les dits acides gras étant choisis parmi les acides stéariques, palmitiques, cetostéariques, arachidiques, béhéniques ;
    - les éthers d'alcool gras et de sucres, les alcools gras étant les alcools stéariques, palmitiques, cetostéariques, arachidiques, béhéniques ; lesdits sucres étant par exemple des sucres réducteurs, et plus particulièrement le glucose, le xylose, l'arabinose, le mannose, ou le saccharose ;
    - les sels divalents des acides gras tels que les sels de magnésium, de zinc ou de calcium des acides stéariques, palmitiques, cetostéariques, arachidiques, béhéniques ;
    - les alcools gras condensés avec de l'oxyde de propylène et/ou de l'oxyde de butylène ;
    - les copolymères d'alkoxydes blocs (éthylène, propylène, butylène...), riches en oxyde de propylène ou de butylène.

Une composition telle que définie ci-dessus caractérisée en ce que ledit au moins un tensioactif lipophile est un stéarate de sorbitan.

- Une composition telle que définie ci-dessus caractérisée en ce qu'elle comprend en outre, jusqu'à 100% de sa masse, une quantité d'additifs tels qu'un parfum, un arôme, un appétant, un colorant, un pigment, ou un diluant solide à une température inférieure ou égale à 20°C tel qu'une huile végétale hydrogénée comme les huiles de soja, de ricin, de colza ou de palme hydrogénée, un beurre de karité, une cire comme la cire d'abeille ou la cire de carnauba,

La présente invention a aussi pour objet :

- Une formulation galénique à libération contrôlée comprenant pour 100% de sa masse :
  - de 20% à 95% massique d'au moins une substance active pharmaceutique, alimentaire ou vétérinaire ; et
  - de 5% à 80% massique d'une composition telle que définie ci-dessus.
- Une formulation telle que définie ci-dessus, caractérisée en ce qu'elle comprend pour 100% de sa masse :
  - de 50% à 90% massique d'au moins une substance active pharmaceutique, alimentaire ou vétérinaire ; et
  - de 10% à 50% massique d'une composition telle que définie ci-dessus.
- Une formulation telle que définie ci-dessus, caractérisée en ce que la substance active est choisie parmi les antiulcéreux, antidiabétiques, anticoagulants, antithrombiques, hypolipémiants, antiarythmiques, vasodilatateurs, antiangineux, antihypertenseurs, vasoprotecteurs, antibiotiques, antifongiques, antiviraux, anticancéreux, anti-inflammatoires, analgésiques, antiépileptiques antiparkinsoniens, neuroleptiques , hypnotiques, anxiolytiques, psychostimulants, antimigraineux, antidépresseurs, antitussifs, antihistaminiques ou antiallergiques.
- Une formulation telle que définie ci-dessus, caractérisée en ce que la substance active est choisie parmi les composés suivants : metformine, acide acétylsalicylique, pentoxifylline, prazosine, acyclovir, nifedipine, diltiazem, naproxen, ibuprofen, flurbiprofen, ketoprofen, fenoprofen, indométhacine, diclofenac, fentiazac, oestradiol valérate, métoprolol, sulpiride, captopril, cimetidine, zidovudine, nicardipine, terfenadine, atenolol, salbutamol, carbamazepine, ranitidine, énalapril, simvastatine, fluoxétine, alprazolam, famotidine, ganciclovir, famciclovir, spironolactone, 5-asa, quinidine, morphine, pentazocine, paracétamol, oméprazole, métoclopramide et leurs mélanges.
- Une formulation telle que définie ci-dessus, caractérisée en ce que la substance active est choisie parmi une liste actifs nutritionnels fortifiants, antioxydants, stimulants tels que des sels minéraux notamment des sels de fer, des vitamines, de l'urée, de la caféine.

La présente invention a également pour objet l'utilisation d'une composition telle que définie précédemment, pour l'encapsulation d'une substance active pharmaceutique, alimentaire ou vétérinaire dans une formulation galénique à libération contrôlée.

Enfin, la présente invention a pour objet un procédé de préparation d'une formulation telle que définie ci-dessus, comprenant les étapes suivantes :

a) on chauffe une composition telle que définie précédemment à une température supérieure ou égale à 40°C pour faire fondre ladite composition ;
b) mélange de la substance active pharmaceutique, alimentaire ou vétérinaire dans la composition fondue issue de l'étape a) ;
c) refroidissement du mélange afin d'obtenir l'encapsulation ou l'enrobage de ladite substance active ;

caractérisé en ce qu'on choisit les valeurs de HLB des tensioactifs et leurs proportions massiques dans la composition introduite à l'étape a) en fonction de la durée ciblée de la libération ultérieure de ladite substance active.

Par composition cireuse on entend une composition insoluble ou peu soluble dans l'eau et solide à température ambiante (c'est-à-dire 20°C).

Le choix du rapport massique entre les deux types de tensioactifs permet de maitriser la vitesse de dissolution de la pellicule cireuse déposée sur un noyau dans le cas d'une utilisation par « hot melt coating » ou la vitesse de dissolution de la microbille formée par « prilling ». La vitesse de libération des principes actifs contenus dans les noyaux ou dans les microbilles est directement liée aux vitesses de dissolution des pellicules ou microbilles cireuses obtenues selon ces techniques.

L'avantage de la composition objet de la présente invention est le contrôle très précis des vitesses de libération d'une molécule active dans le système digestif humain ou animal, tout en masquant le goût de la dite molécule. On peut même obtenir un effet retard permettant une libération nulle pendant le transit stomacal pour ne commencer à libérer l'actif que dans l'intestin. On va pouvoir « moduler » cette libération en fonction de l'endroit de l'intestin que l'on a comme cible.

Cette régulation « fine » de la libération de l'actif dans le système digestif se fait grâce à un mélange de plusieurs tensioactifs solides à température ambiante et choisis en fonction de leur valeur HLB respective. Les tensioactifs hydrophiles sont caractérisés par une valeur de HLB supérieure ou égale à 10. Les tensioactifs lipophiles ont une valeur de HLB supérieure ou égale à 1 et inférieure à 10.

Les compositions cireuses selon l'invention comprennent généralement un minimum de deux tensioactifs, mais il se peut que des compositions intègrent plus de tensioactifs différents. C'est le mélange de ces tensioactifs, faisant varier à la fois leurs compositions pour obtenir des mélanges de HLB bien précis et leurs proportions dans le mélange qui va permettre un contrôle très fin de la libération de l'actif choisi tout en conservant les propriétés de masquage de goût.

Selon un mode particulier de l'invention, la propriété de gastrorésistance n'est pas obtenue, comme dans la plupart des publications, par l'utilisation de polymères acryliques ou de succinates acétates mais par le choix judicieux de tensioactifs ayant une valeur HLB variant avec le pH.

Parmi les tensioactifs lipophiles utilisables, on peut citer :
Les esters d'acides gras solides à température ambiante, et de sucres ; lesdits acides gras étant choisis parmi les acides stéariques, palmitiques, cetostéariques, arachidiques, béhéniques ; les dits sucres étant par exemple du glucose, du sorbitol, du mannitose, du mannitol, du saccharose, du mannose, du xylitol ou du xylose.

On peut citer plus particulièrement les ester de sorbitol et d'acide palmitique commercialisés sous le nom de marque Montane™40, les esters de sorbitol et d'acide stéarique commercialisés sous les noms de marque Montane™60 et Montane™65. Les esters d'acides gras et de glycérol, les dits acides gras étant choisis parmi les acides stéariques, palmitiques, cetostéariques, arachidiques, béhéniques.

Les éthers d'alcool gras et de sucres. Les alcools gras particulièrement utiles étant les alcools stéariques, palmitiques, cetostéariques, arachidiques, béhéniques ; lesdits sucres étant par exemple des sucres réducteurs, et plus particulièrement le glucose, le xylose, l'arabinose, le mannose, ou le saccharose

Les sels divalents des acides gras tels que les sels de magnésium, de zinc ou de calcium des acides stéariques, palmitiques, cetostéariques, arachidique, béhéniques.

Les alcools gras solides à température ambiante (20°C) condensés avec de l'oxyde de propylène et/ou de l'oxyde de butylène.

Les copolymères blocs d'alkoxydes (ethylène , propylène , butylène...), riches en oxyde de propylène et/ou de butylène.

Parmi les tensioactifs hydrophiles utilisables, il y a :

- Les dérivés éthoxylés des composés mentionnés ci-dessus ayant un nombre de motifs d'oxyde d'éthylène supérieur ou égal à cinq, de préférence supérieur à dix, de préférence égal à vingt. On peut citer plus particulièrement les dérivés éthoxylés ayant un nombre de motifs d'oxyde d'éthylène supérieur ou égal à cinq, de préférence supérieur ou égal à dix, de préférence égal à vingt, des esters d'acides gras solides à température ambiante et de sucres ; lesdits acides gras étant choisis parmi les acides stéariques, palmitiques, cetostéariques, arachidique, béhéniques ; les dits sucres étant par exemple du sorbitol, du mannitol ou du xylitol. On peut citer encore plus particulièrement les esters d'acide stéariques et de sorbitol éthoxylés à 20 moles d'oxyde d'éthylène et commercialisés sous les noms de marque Montanox™60 et Montanox™65, les esters d'acide palmitique et de sorbitol éthoxylés à 20 moles d'oxyde d'éthylène et commercialisés sous les noms de marque Montanox™40 les esters d'acide stéariques et de sorbitol éthoxylés à 20 moles d'oxyde d'éthylène et commercialisés sous les noms de marque Montanox™60 et Montanox™65.

- Les esters de polyglycérol formés par condensation d'un acide gras, choisi parmi les acides stéariques, palmitiques, cetostéariques, arachidiques, béhéniques, et d'un polyglycérol comprenant au moins trois motifs de glycérol.
- Les alcools gras alkoxylés formés par condensation d'alcools solides à température ambiante et d'oxyde d'éthylene et/ou d'oxyde de propylène, riches en oxyde d'éthylène.
- Les copolymères blocs d'alkoxydes (éthylène, propylène, butylène...), riches en oxyde d'éthylène.
- Les tensioactifs dont la valeur HLB varie avec le pH peuvent être choisis dans les familles suivantes :

  - acides gras (lipophiles à pH inférieur à environ 4 et hydrophiles à pH supérieurs à environ 5). Les acides gras préférés sont les acides stéariques, palmitiques, cetostéariques, arachidiques, béhéniques.
  - Sels monovalents de sodium, potassium d'acides gras tels que les acides stéariques, palmitiques, cetostéariques, arachidiques, béhéniques.
  - Les tensioactifs résultant de la réaction de N-acylation entre un chlorure d'acide gras ou un ester méthylique d'acide gras ou un acide gras et au moins un acide aminé ou au moins un peptide ou au moins un hydrolysat de protéine ou au moins une protéine ou au moins un hydrolysat de peptide, plus particulièrement les agents tensioactifs résultant de la réaction de N-acylation entre un chlorure d'acide gras ou un ester méthylique d'acide gras ou un acide gras et au moins un acide aminé, encore appelés N-acylat d'acides aminés ou lipoaminoacides.

Les HLB sont des grandeurs additives. Une composition constituée de deux tensioactifs de HLB respectives H1 et H2 en proportions pondérales respectives x1 et x2 sera donc caractérisée par une $HLB_c$ calculable par la formule :

$$HLB_c = x_1 * H_1 + x_2 * H_2$$

La présente invention a aussi pour objet une méthode consistant à disperser, suspendre ou émulsifier la matière dont on veut masquer le goût et contrôler la libération dans un mélange de tensioactifs fondus et de laisser tomber goutte à goutte par des buses ce mélange dans un courant d'azote liquide ou d'air refroidi à l'azote liquide. Le diamètre des buses sera choisi de telle manière qu'il soit environ la moitié de celui des microbilles désirées. Ainsi, on choisira de préférence des buses de diamètre compris entre 50 μm et 1000 μm, de préférence entre 100 μm et 500 μm.

Une variante de ce procédé consiste à amener le mélange cireux fondu comprenant les tensioactifs et la substance active à la surface d'un disque tournant à vitesse contrôlée ; la vitesse du disque provoquant par centrifugation la formation de microgouttelettes qui vont se solidifier et donner des microbilles solides lors de leur chute dans un courant d'air froid ou dans de l'azote liquide.

Une autre façon de mettre en œuvre l'invention est de déposer sur la matière active une couche de ce mélange de tensioactifs fondus au moyen d'une table de refroidissement équipée de rideaux de dispersion ou de buses de vaporisation. On peut également utiliser une turbine de pelliculage pour cette opération.

Dans les deux cas, la matière fondue contenant l'actif en suspension (cas d'une poudre), dissout (cas d'un liquide hydrophobe) ou émulsifié (cas d'un liquide hydrophile), va se solidifier immédiatement et enrober ou encapsuler la matière active. Ces deux technologies vont donner des granulés ou des billes de matière active encapsulée.

On peut améliorer ces technologies de la manière suivante : en faisant vibrer les buses, on peut obtenir des billes de même taille (granulométrie monodisperse) qui rend encore plus facile leur utilisation éventuelle dans des tablettes ou des comprimés.

Mais on peut aussi incorporer aux mélanges de tensioactifs des arômes qui vont augmenter l'attractivité du produit pour les animaux ou les jeunes enfants.

Exemple 1 : Libération contrôlée et masquage de goût d'une poudre métallique hydrophile.

Dans cet exemple, la substance active à encapsuler est une poudre hydrophile constituée d'un sulfate métallique. Elle est utilisée pour rétablir l'équilibre en métal lors d'une carence notamment chez les jeunes enfants.

Son goût est un goût très astringent typique des sels de métaux et surtout son caractère hydrophile est tel que le produit se dissout en moins d'une heure dans l'estomac, rendant impossible une administration continue dans la journée et augmentant de façon brutale la teneur en métal dans le sang.

Ladite poudre hydrophile est par exemple constituée d'un sulfate de calcium, de manganèse, de zinc ou de fer.

Description des travaux :

Une seringue de 5 ml en verre équipée d'une aiguille 20 G afin de faire goutter des billes du mélange de tensioactifs / poudre métallique fondu à 75°C dans de l'eau froide est utilisée. Puis le mélange fondu est maintenu dans la seringue.

On arrive à préparer des billes d'environ 2 mm de diamètre contenant, pour 100% de leur masse, 50% massique de poudre hydrophile et 50% massique d'un mélange Montane 60/ Montanox 60 dans un ratio massique intime de 90/10.

Une évaluation de la cinétique de libération a été réalisée par suivi de conductivité. On obtient un résultat conforme à la demande (80% à 100% de substance active libérée entre six heures et huit heures).

Deux échantillons de 30 g de billes sont préparés, et le tableau ci-dessous indique leur composition massique pour 100% de leur masse.

| Poudre hydrophile | 50% | 50% |
|---|---|---|
| Montane 60* | 50% | 45% |

(suite)

| Montanox 60** | | 5% |
|---|---|---|
| *Monostéarate de sorbitan fabriqué par SEPPIC<br>**Monostéarate de sorbitan éthoxylé à 20 moles d'oxyde d'éthylène fabriqué par SEPPIC. | | |

Evaluation de la vitesse de libération de l'ion métallique:

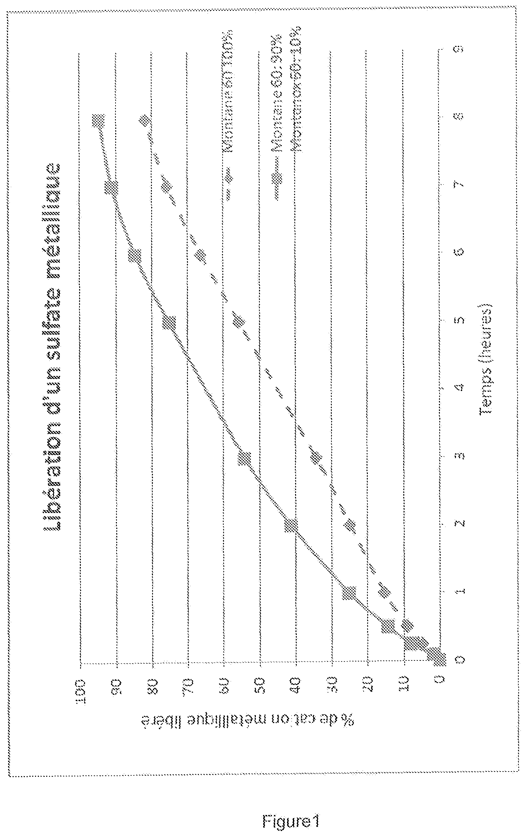

1 g de billes est dispersé dans 100 g d'eau. L'évolution de la conductivité dans le temps est suivie. Les résultats sont illustrés à la figure 1.
Témoin : solution à 0,5 g de poudre seule dans 100 g d'eau : 3560 mS/cm.

Exemple 2 : Encapsulation et masquage de goût d'un actif vétérinaire :

Dans cet exemple l'actif à protéger et encapsuler est un antiparasitaire vétérinaire (API). Ce principe actif, administré principalement par voie entérale, est très amer, il est détecté facilement par les félins dont l'odorat et le goût sont très sensibles. Il se présente sous forme d'une poudre blanche de point de fusion élevé (135°C) et très peu soluble dans l'eau (200mg/l à 20°C).
Comme antiparasitaire vétérinaire, on peut citre par exemple des insectifuges à usage externe : huiles essentielles comme le géraniol ou extrait de lavandin. Des insecticides à usage interne : Fiproli, avermectine.
D'autres exemples sont les vermifuges et acaricides comme le febantel, le niclosamide, le pyrantel. Un très bon exemple est le praziquantel.
L'encapsulation a pour but le masquage de gout du principe actif pendant environ 15 minutes. Cela implique une très faible libération de l'API (une quantité n'excédant pas 10% massique de l'API) pendant cette durée minimale de 15 minutes cet intervalle de temps, mais il devra être totalement libéré, au bout d'une durée de une à deux heures après administration, dans l'estomac de l'animal à pH 4 à 5.
La concentration en principe actif dans la matrice d'encapsulation devra approcher 40% massique.
Différents mélanges de tensioactifs hydrophiles et lipophiles ont été évalués, en association avec 40% massique d'actif. Les formulations ont pour composition, pour 100% de leurs masses :

Principe actif: 40%
Tensioactif lipophile : 30% - 60%
Tensioactif hydrophile : 0% - 30%

Tableau des tensioactifs utilisés pour les essais

| Nature | Nom commercial | HLB | Caractère |
|---|---|---|---|
| Di béhénate de glycérol | Compritol 888 (Gattefossé) | 1,8 | lipophile |
| Mono béhenate de glycérol | Essai Seppic | 2 | lipophile |
| Stéarate de sorbitan | Montane 60 (Seppic) | 4,7 | lipophile |
| Alcool cétostéarylique éthoxylé (8 OE) | Simulsol 58 PHA (Seppic) | 15,7 | hydrophile |
| Stéarate de sorbitan éthoxylé (20 OE) | Montanox 60 (Seppic) | 14,9 | hydrophile |

Protocole expérimental pour la fabrication des microbilles.

Tous les ustensiles utilisés sont mis à l'étuve à 100°C. Les excipients sont fondus à l'étuve à 100°C. Dans un réacteur de 500 mL chaud mis au bain marie sur un agitateur magnétique thermostaté à 145°C. Les excipients fondus sont introduits sous agitation. La substance active est ajoutée et homogénéisée jusqu'a obtenir un liquide huileux d'aspect jaunâtre ou marron. Le mélange fondu est refroidi puis broyé et tamisé pour obtenir des microbilles solides.
La granulométrie des microbilles est caractérisée en diffraction laser par leur Dv90 (taille maximale de 90% des particules) et leur Dv50 (diamètre moyen des particules), à l'aide d'un granulomètre MALVERN Mastersizer.

Protocole expérimental pour la libération de l'actif.

Le protocole de dissolution décrit dans la pharmacopée américaine a été suivi. 100g de microbilles sont dispersés dans un litre de solution aqueuse à pH 5, maintenue à 37°C. Des prélèvements sont effectués périodiquement et dosés par HPLC pour leur concentration en principe actif.

Composition et caractérisation des microbilles réalisées.

| Référence | Composition pondérale | | | HLB microbille | Granulométrie (μm) | |
|---|---|---|---|---|---|---|
| | Principe actif | TA lipophile | TA hydrophile | | Dv90 | DV50 |
| 1 | 40% | Di béhénate de glycérol 60% | 0 | 1,8 | 900 | ND |
| 2 | 40% | Di béhénate de glycérol 60% | 0 | 1,8 | 493 | ND |
| 3 | 50% | Di béhénate de glycérol 50% | 0 | 1,8 | 872 | ND |
| 4 | 40% | Di béhénate de glycérol 50% | Alcool cetostearylique 8 OE 10% | 4,3 | 494 | 250 |
| 5 | 40% | Di béhénate de glycérol 40% | Alcool cetostearylique 8 OE 20% | 6,6 | 514 | 266 |
| 6 | 40% | Di béhénate de glycérol 30% | Alcool cetostearylique 8 OE 30% | 8,9 | 496 | 248 |
| 7 | 40% | Monobéhénate de glycérol 60% | 0 | 2 | 555 | 284 |
| 8 | 40% | Di béhénate de glycérol 40% | Stéarate de sorbitan 20 OE 20% | 6,3 | 498 | 252 |

Résultats de libération de l'actif des différents essais réalisés.

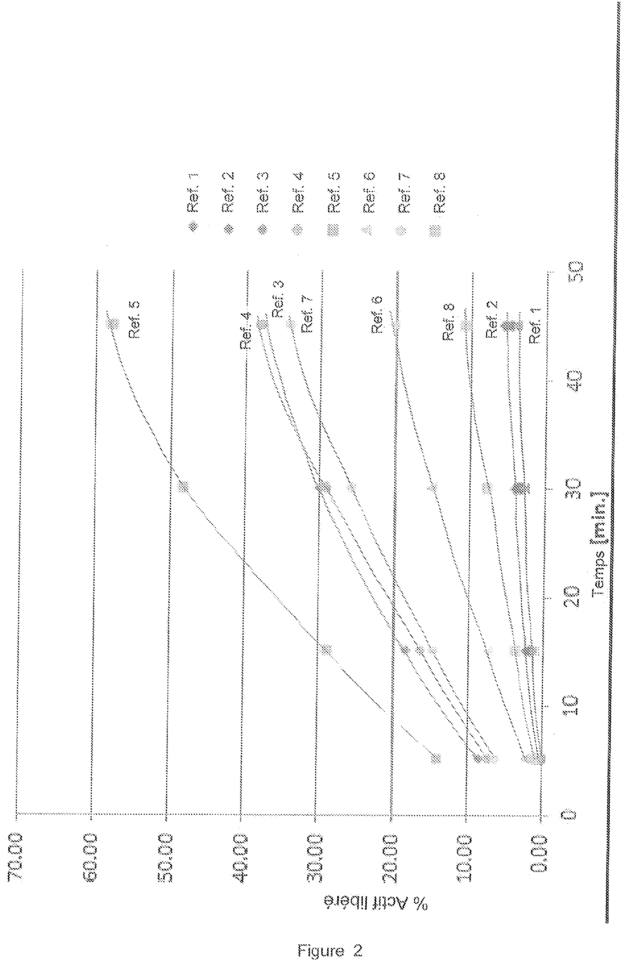

On peut voir dans les courbes de la figure 2 que les différentes compositions testées modifient totalement les profils de libération du principe actif.

Les essais 1 et 2, de compositions identiques, donnent des profils de libération superposables, montrant la reproductibilité et la robustesse du procédé. Ils permettent de ne libérer qu'une partie infime de l'actif (jusqu'à 4% massique en 30 minutes) et donc un masquage de goût efficace. L'essai 3 de même composition qualitative mais plus chargé en principe actif présente un profil de libération légèrement plus rapide, conformément aux principes des lois de diffusion.

Les courbes permettent aussi de constater que, plus la valeur HLB de la microbille est élevée, plus la libération de l'actif est rapide.

Les compositions ne contenant pas de tensioactif hydrophile (et qui ne correspondent donc pas à l'invention) comme les 7, 1, 2 et 3 libèrent effectivement très peu d'actif dans le premier quart d'heure mais n'en libèrent pas beaucoup plus ensuite. L'actif reste donc en grande partie piégé dans la capsule et n'est plus efficace. La composition ayant la valeur HLB la plus élevée (essai 6) libère pratiquement tout l'actif en une heure mais aussi beaucoup d'actif en quinze minutes, ce qui lui confère des propriétés de masquage de gout limitées. Il s'agit donc de trouver le bon compromis, ce qui est le cas avec des compositions telles que les 4, 5 et 8. Les compositions visées sont celles qui libèrent moins de 10% d'actif en 15 minutes pour avoir l'effet masquage de gout et plus de 30% d'actif en 45 minutes pour avoir une efficacité du principe actif dans le tube digestif.

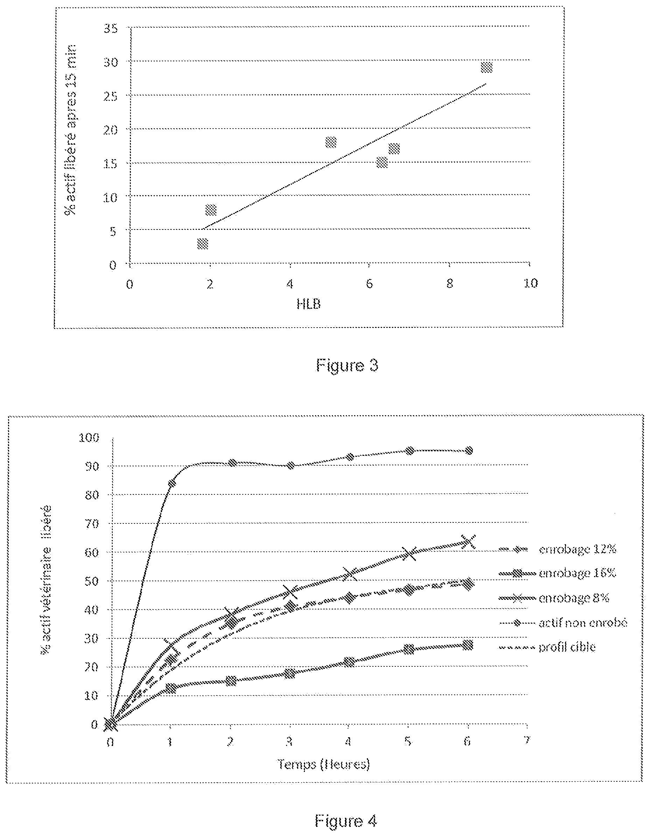

Cette corrélation peut aussi être visualisée sur le graphique de la figure 3 montrant le pourcentage massique d'actif libéré au bout de 15 minutes en fonction de la valeur du HLB des microbilles.

Lors de la réalisation du produit fini, un appétant pour chats été ajouté à la solution d'encapsulation par prilling de tensioactifs pour accroitre encore l'attractivité du produit pour les animaux.

L'addition de l'appétant à la dose de 0,5% n'a pas modifié ni les conditions de « prilling » ni les profils de libération.

Exemple 3 : Cas d'un additif vétérinaire pour ruminants :

On s'intéresse dans cet exemple à un principe actif hydrophile pour l'alimentation des bovins, soluble en trente secondes dans le rumen du ruminant.

L'objectif est de l'encapsuler de façon à ce qu'il se libère dans le rumen de bovins de façon progressive en environ six à huit heures.

On cherche à réaliser l'encapsulation de cet actif dans des mélanges impliquant au moins deux tensioactifs ayant des valeurs HLB différentes pour obtenir un profil de libération cible, caractérisé par la libération de environ 20% de l'actif administré après une heure, 40% après quatre heures et 50% après six heures.

La composition retenue comprend :

- Deux tensioactifs: Stéarate de sorbitan - tensioactif lipophile de HLB 4,7 et Stéarate de sorbitan ethoxylé (20 OE) - tensioactif hydrophile de HLB 15.
- Un diluant : huile de palme hydrogénée

Le HLB de la composition est de 5,1 ; la proportion massique de ses constituants étant indiquée ci-dessous pour 100% de la masse de ladite composition :

| | |
|---|---|
| Huile de palme hydrogénée : | 74% |
| Stéarate de sorbitan : | 25% |
| Oléate de sorbitan Ethoxylé 20 OE : | 1%. |

L'encapsulation de l'actif est réalisée en pulvérisant la composition fondue à 70°C sur l'actif dans une turbine de pelliculage dont la température est maintenue voisine de 50°C. La pulvérisation s'effectue au moyen d'un appareil de marque Accucoat® muni d'un pistolet de type Airless.

La dissolution de l'actif est réalisée dans un banc de dissolution conforme à la pharmacopée européenne. 2,5g de microcapsules sont dispersés dans un litre d'une solution aqueuse à pH 6 maintenue à 37°C avec une vitesse d'agitation de 100 tours/minute. La libération de l'actif est suivie par prélèvements réguliers objet de dosage dudit actif par spectrophotométrie à 420 nm.

Les courbes de la figure 4 montrent que l'on peut atteindre le profil de libération cible en encapsulant 88% massique d'actif dans 12% de la composition retenue et que le profil de libération peut être ajusté en fonction de la quantité de la composition déposée sur l'actif : plus la quantité déposée est élevée, plus la dissolution de l'actif est lente.

Exemple 4 : Effet du HLB de la composition d'encapsulation sur la libération d'un additif vétérinaire pour ruminants.

Différentes compositions de tensioactifs hydrophiles et lipophiles dilués dans une huile végétale hydrogénée ont été étudiées pour encapsuler l'actif vétérinaire de l'exemple 3.

La substance active est par exemple un acide aminé tel que la choline, l'urée, les vitamines (par exemple la vitamine C), ou un sel minéral (de calcium ou de fer).

Le stéarate de sorbitan est un tensioactif lipophile de HLB 4,7. L'oléate de sorbitan ethoxylé à 20 moles d'oxyde d'éthylène est un tensioactif hydrophile de HLB 15.

Les cinétiques de libération de l'actif ont été mesurées dans les mêmes conditions que précédemment décrites à l'exemple 3.

Les compositions pondérales des phases grasses mises en œuvre pour encapsuler l'actif sont données ci-dessous, pour 100% de la masse des compositions formées.

| Référence | Huile végétale hydrogénée (%) | Stéarate de sorbitan (%) | Oléate de sorbitan 20 OE (%) | HLB de la composition |
|---|---|---|---|---|
| 9 | 47,5 | 47,5 | 5 | 5,7 |
| 10 | 50 | 50 | 0 | 4,7 |

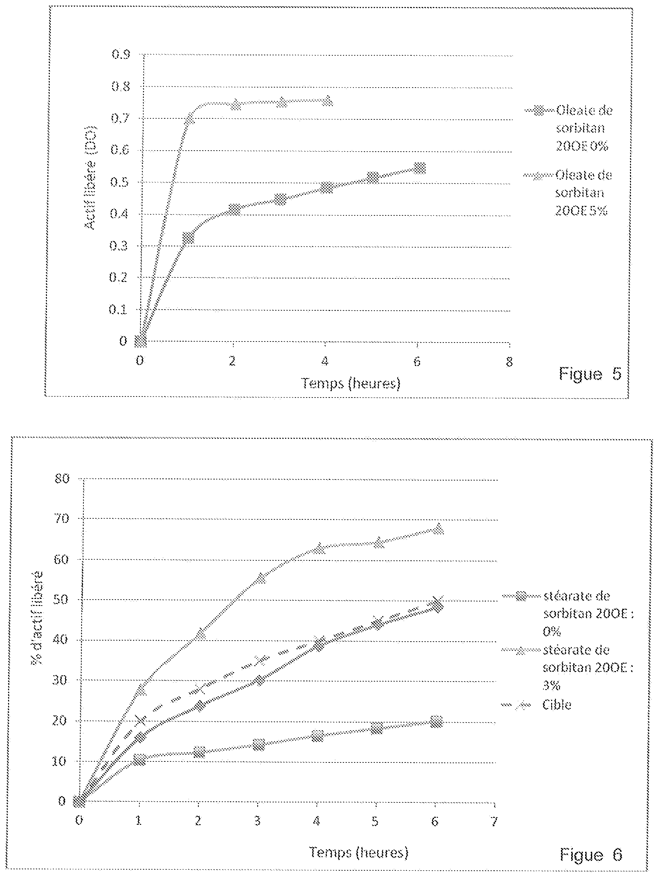

Les courbes de la figure 5 montrent les cinétiques de libération de l'actif réalisées dans les mêmes conditions expérimentales que celles décrites plus haut. On constate à nouveau que la vitesse de libération augmente avec le HLB de la composition enrobante.

Dans une autre série d'essais, nous avons formulé des capsules comprenant, pour 100% de leur masse, 90% massique d'actif et 10% massique d'enrobage ayant, pour 100% massique dudit enrobage, les compositions suivantes.

| Référence | Huile végétale hydrogénée (%) | Stéarate de sorbitan (%) | Oléate de sorbitan 20 OE (%) | HLB de la composition |
|---|---|---|---|---|
| 11 | 75 | 25 | 0 | 4,7 |
| 12 | 74,3 | 24,7 | 1 | 5,1 |
| 13 | 72,8 | 24,2 | 3 | 5,8 |

Les courbes de libération de l'actif présentées à la figure 6 montrent que l'on peut atteindre un profil cible en ajustant la concentration en tensioactif hydrophile à 1% dans la composition d'enrobage.

Exemple 5 : microbilles gastrorésistantes comprenant deux tensioactifs.

L'objectif est la mise au point d'une composition se présentant sous la forme de microbilles et contenant un principe actif pharmaceutique, le diclofénac sodique, qui ne se dissolve pas dans l'estomac à pH acide mais qui permette la libération du principe actif dans l'intestin (pour une valeur du pH supérieure ou égale à 5 et inférieure ou égale à 8).

Pour atteindre cet objectif, nous avons formulé une composition comprenant une association de deux tensioactifs : un tensioactif lipophile (stéarate de sorbitan, HLB = 4,7) et un tensioactif dont la valeur HLB change en fonction du pH. L'acide stéarique est lipophile en milieu acide et hydrophile en milieu neutre ou basique (HLB voisin de 20).

Le mélange cireux (stéarate de sorbitan / Acide Stéarique) est chauffé à 85°C grâce à un bain-marie. Le diclofénac sodique est ajouté sous agitation avec une défloculeuse/Rayneri.

Lorsque le mélange est homogène, il est placé dans la machine de prilling.

Protocole expérimental :

On obtient une formule liquide à 85°C qui fige à température ambiante dont la composition pondérale est pour 100% de sa masse :

- 21% Stéarate de sorbitan (Montane™ 60),
- 49% Acide Stéarique,
- 30% Diclofénac sodique.

La formule fondue est introduite dans la cuve d'un dispositif de prilling à disque tournant de marque SPRAI.

Les paramètres opératoires pour la formation des microbilles sont les suivants :

- Température de chauffe de la cuve : 85°C,
- Température de chauffe du tuyau d'alimentation du disque : 85°C,
- Vitesse de rotation du disque : 50 tours/s,
- Pression dans la cuve : 0,6 bar.

Dans ces conditions, on obtient des microbilles sphériques de diamètre inférieur à 500µm.

La libération du diclofénac est mesurée dans un banc de dissolution conforme à la pharmacopée européenne :

- Milieu de libération : solution de HCl à pH = 1 (750 mL) pendant deux heures puis ajout de 250 mL de tampon phosphate puis ajustement du pH à 6,8 avec une solution de NaOH à 5%. Billes laissées à pH=6,8 pendant quatre heures,
- 150g de billes placées directement dans le milieu de dissolution,
- Quantité du milieu de libération par bol : 1L,
- Quantité de milieu prélevée à chaque temps : 3 mL,
- Température du milieu de libération : 37°C.

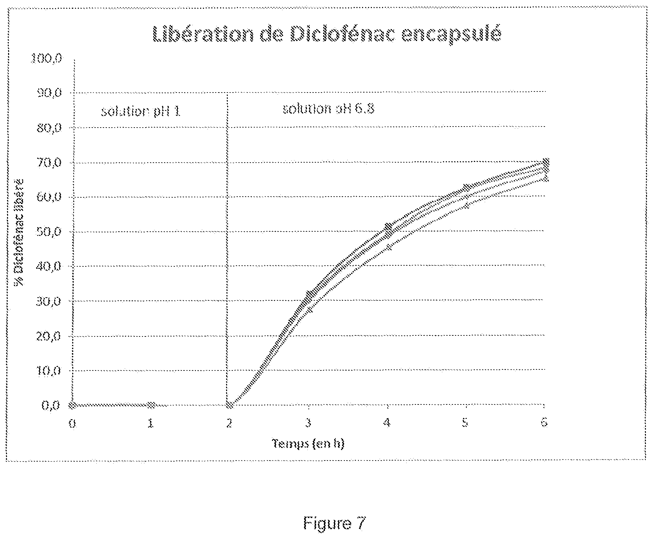

La cinétique de libération obtenue est donnée sur la courbe de la figure 7. Elle montre que l'actif ne se libère pas des microbilles en milieu acide mais se dissout rapidement dès que le pH du milieu de dissolution est augmenté à une valeur de 6,8. La reproductibilité des cinétiques de libération évaluée sur quatre essais identiques est bonne.

## Revendications

**1.** Composition insoluble dans l'eau, d'aspect solide à une température inférieure ou égale à 20°C, comprenant pour 100% de sa masse :

- X1% massique d'au moins un tensioactif lipophile ayant une valeur HLB, H1, supérieure ou égale à 1 et inférieure à 10 ;
- X2% massique d'au moins un tensioactif hydrophile ayant une valeur HLB, H2, supérieure ou égale à 10 et inférieure ou égale à 20 ;

**caractérisée par le fait que** son HLB = X1.H1 + X2.H2, X1 et X2 variant de 2 à 60 et **caractérisée en ce qu'**elle est exempte de polymère acrylique et/ou de succinate acétate, et **caractérisée en ce que** :

- ledit au moins un tensioactif lipophile est un stéarate de sorbitan,et
- ledit tensioactif hydrophile est choisi parmi les esters de sorbitan éthoxylés, les alcools ou acides éthoxylés, les esters de polyglycérol, les éthers de glucose, les copolymères blocs d'oxydes d'éthylène et de propylène.

**2.** Composition selon la revendication précédente **caractérisée en ce que** H1 est supérieure ou égale à 3,4 et inférieure ou égale à 6 et H2 est supérieure ou égale 14 et inférieure ou égale à 16.

**3.** Composition selon l'une des revendications précédentes **caractérisée en ce qu'**elle comprend de deux à cinq tensioactifs ayant chacun un HLB, Hi, et une proportion massique Xi tels que la valeur HLB de ladite composition est égale à $\sum$ Xi.Hi, avec i compris entre 2 et 5, xi compris entre 2% et 60% et $\sum$ Xi $\leq$ 100%.

**4.** Composition selon l'une des revendications précédentes **caractérisée en ce que** ledit au moins un tensioactif lipophile est choisi parmi

- les esters d'acides gras et de sucres ; les dits acides gras étant choisis parmi les acides stéariques, palmitiques, cetostéariques, arachidiques, béhéniques ; les dits sucres étant par exemple du glucose, du sorbitol, du mannitose, du mannitol, du saccharose, du mannose, du xylitol ou du xylose;
- les esters d'acides gras et de glycérol, les dits acides gras étant choisis parmi les acides stéariques, palmitiques, cetostéariques, arachidiques, béhéniques ;
- les éthers d'alcool gras et de sucres, les alcools gras étant les alcools stéariques, palmitiques, cetostéariques, arachidiques, béhéniques ; lesdits sucres étant par exemple des sucres réducteurs, et plus particulièrement le glucose, le xylose, l'arabinose, le mannose, ou le saccharose ;
- les sels divalents des acides gras tels que les sels de magnésium, de zinc ou de calcium des acides stéariques, palmitiques, cetostéariques, arachidiques, béhéniques ;
- les alcools gras condensés avec de l'oxyde de propylène et/ou de l'oxyde de butylène ;
- les copolymères d'alkoxydes blocs (éthylène, propylène, butylène...), riches en oxyde de propylène ou de butylène.

**5.** Composition selon l'une des revendications précédentes **caractérisée en ce qu'**elle comprend en outre, jusqu'à 100% de sa masse, une quantité d'additifs tels qu'un parfum, un arôme, un appétant, un colorant, un pigment, ou un diluant solide à une température inférieure ou égale à 20°C tel qu'une huile végétale hydrogénée comme les huiles de soja, de ricin, de colza ou de palme hydrogénée, un beurre de karité, une cire comme la cire d'abeille ou la cire de carnauba,

**6.** Formulation galénique à libération contrôlée comprenant pour 100% de sa masse :

- de 20% à 95% massique d'au moins une substance active pharmaceutique, alimentaire ou vétérinaire ; et
- de 5% à 80% massique d'une composition telle que définie à l'une des revendications 1 à 5.

**7.** Formulation selon la revendication précédente, **caractérisée en ce qu'**elle comprend pour 100% de sa masse :

- de 50% à 90% massique d'au moins une substance active pharmaceutique, alimentaire ou vétérinaire ; et
- de 10% à 50% massique d'une composition telle que définie à l'une des revendications 1 à 6.

**8.** Formulation selon l'une des revendications 6 ou 7, **caractérisée en ce que** la substance active est choisie parmi les antiulcéreux, antidiabétiques, anticoagulants, antithrombiques, hypolipémiants, antiarythmiques, vasodilatateurs, antiangineux, antihypertenseurs, vasoprotecteurs, antibiotiques, antifongiques, antiviraux, anticancéreux, anti-inflammatoires, analgésiques, antiépileptiques, antiparkinsoniens, neuroleptiques, hypnotiques, anxiolytiques, psychostimulants, antimigraineux, antidépresseurs, antitussifs, antihistaminiques ou antiallergiques.

**9.** Formulation selon la revendication précédente **caractérisée en ce que** la substance active est choisie parmi les composés suivants : metformine, acide acétylsalicylique, pentoxifylline, prazosine, acyclovir, nifedipine, diltiazem, naproxen, ibuprofen, flurbiprofen, ketoprofen, fenoprofen, indométhacine, diclofenac, fentiazac, oestradiol valérate, métoprolol, sulpiride, captopril, cimetidine, zidovudine, nicardipine, terfenadine, atenolol, salbutamol, carbamazepine, ranitidine, énalapril, simvastatine, fluoxétine, alprazolam, famotidine, ganciclovir, famciclovir, spironolactone, 5-asa, quinidine, morphine, pentazocine, paracétamol, oméprazole, métoclopramide et leurs mélanges.

**10.** Formulation selon l'une des revendications 6 ou 7, **caractérisée en ce que** la substance active est choisie parmi une liste actifs nutritionnels fortifiants, antioxydants, stimulants tels que des sels minéraux notamment des sels de fer, des vitamines, de l'urée, de la caféine.

**11.** Utilisation d'une composition telle que définie à l'une des revendications 1 à 5 pour l'encapsulation d'une substance active pharmaceutique, alimentaire ou vétérinaire dans une formulation galénique à libération contrôlée.

**12.** Procédé de préparation d'une formulation telle que définie à l'une des revendications 6 à 9 comprenant les étapes suivantes :

a) on chauffe une composition telle que définie à l'une des revendications 1 à 5 à une température supérieure ou égale à 40°C pour faire fondre ladite composition ;
b) mélange de la substance active pharmaceutique, alimentaire ou vétérinaire dans la composition fondue issue de l'étape a) ;
c) refroidissement du mélange afin d'obtenir l'encapsulation ou l'enrobage de ladite substance active ;

**caractérisé en ce qu'**on choisit les valeurs de HLB des tensioactifs et leurs proportions massiques dans la composition introduite à l'étape a) en fonction de la durée ciblée de la libération ultérieure de ladite substance active.

## Patentansprüche

**1.** Wasserunlösliche Zusammensetzung, mit festem Erscheinungsbild bei einer Temperatur von weniger als oder gleich 20 °C, umfassend für 100 % ihrer Masse:

- X1 Masse-% mindestens eines lipophilen Tensids, das einen HLB-Wert, H1, größer als oder gleich 1 und kleiner als 10 aufweist;
- X2 Masse-% mindestens eines hydrophilen Tensids, das einen HLB-Wert, H2, größer als oder gleich 10 und kleiner als oder gleich 20 aufweist;

**gekennzeichnet durch** die Tatsache, dass ihr HLB = X1.H1 + X2·H2, wobei X1 und X2 von 2 bis 60 variieren und **dadurch gekennzeichnet, dass** sie frei von Acrylpolymer und/oder Acetat-Succinat ist, und **dadurch gekennzeichnet, dass**:

- das mindestens eine lipophile Tensid eine Sorbitanstearat ist, und
- das hydrophile Tensid ausgewählt ist aus den ethoxilierten Sorbitanestern, den ethoxilierten Alkoholen oder Säuren, den Polyglycerolestern, den Glucoseestern, den Ethylen- und Propylen-Oxid-Blockcopolymeren.

**2.** Zusammensetzung nach dem vorstehenden Anspruch, **dadurch gekennzeichnet, dass** H1 größer als oder gleich 3,4 und kleiner als oder gleich 6 ist und H2 größer als oder gleich 14 und kleiner als oder gleich 16 ist.

3. Zusammensetzung nach einem der vorstehenden Ansprüche, **dadurch gekennzeichnet, dass** sie zwei bis fünf Tenside umfasst, die jeweils ein HLB, Hi, und einen Massenanteil Xi derart aufweisen, dass der HLB-Wert der Zusammensetzung gleich $\sum Xi \cdot Hi$ ist, wobei i zwischen 2 und 5 liegt, xi zwischen 2% und 60% liegt und $\sum Xi \leq 100$ %.

4. Zusammensetzung nach einem der vorstehenden Ansprüche, **dadurch gekennzeichnet, dass** das mindestens eine lipophile Tensid ausgewählt ist aus

- den Fettsäureestern und Zuckern; wobei die Fettsäuren ausgewählt sind aus den Stearin-, Palmitin-, Cetostearin-, Arachin-, Behensäuren; wobei die Zucker beispielsweise Glucose, Sorbitol, Mannitose, Mannitol, Saccharose, Mannose, Xylitol oder Xylose sind;
- den Fettsäureestern und Glycerin, wobei die Fettsäuren ausgewählt sind aus den Stearin-, Palmitin-, Cetostearin-, Arachin-, Behensäuren;
- den Fettalkoholestern und Zuckern, wobei die Fettalkohole die Stearin-, Palmitin-, Cetostearin-, Arachin-, Behenalkohole sind; wobei die Zucker beispielsweise reduzierende Zucker und insbesondere Glucose, Xylose, Arabinose, Mannose oder Saccharose sind;
- zweiwertigen Salzen der Fettsäuren wie Magnesiumsalzen, Zink oder Kalzium der Stearin-, Palmitin-, Cetostearin-, Arachin-, Behensäuren;
- mit Propylenoxid und/oder mit Butylenoxid kondensierten Fettalkoholen;
- Alkoxyd-Blockcopolymeren (Ethylen, Propylen, Butylen...), reich an Propylen- oder an Butylenoxid.

5. Zusammensetzung nach einem der vorstehenden Ansprüche, **dadurch gekennzeichnet, dass** sie weiter bis zu 100 % ihrer Masse eine Menge an Zusatzstoffen, wie etwa einen Duftstoff, ein Aroma, einen Geschmacksanreger, einen Farbstoff, ein Pigment oder eine bei einer Temperatur von weniger als oder gleich 20 °C festes Verdünnungsmittel wie etwa wasserstoffhaltiges Pflanzenöl, wie Soja-, Ricin-, Rapsöle, oder wasserstoffhaltiges Palmöl, eine Sheabutter, ein Wachs, wie Bienenwachs oder Carnaubawachs, umfasst.

6. Galenische Formulierung zur kontrollierten Freisetzung, umfassend für 100 % ihrer Masse:

- von 20 Masse-% bis 95 Masse-% mindestens eines pharmazeutischen, alimentären oder tierärztlichen Wirkstoffs; und
- von 5 Masse-% bis 80 Masse-% einer Zusammensetzung nach einem der Ansprüche 1 bis 5.

7. Formulierung nach dem vorstehenden Anspruch, **dadurch gekennzeichnet, dass** sie für 100 % ihrer Masse umfasst:

- von 50 Masse-% bis 90 Masse- % mindestens eines pharmazeutischen, alimentären oder tierärztlichen Wirkstoffs; und
- von 10 Masse-% bis 50 Masse-% einer Zusammensetzung nach einem der Ansprüche 1 bis 6.

8. Formulierung nach einem der Ansprüche 6 oder 7, **dadurch gekennzeichnet, dass** der Wirkstoff ausgewählt ist aus den Mitteln gegen Geschwüren, Antidiabetika, Antikoagulanzien, Antithrombotika, Cholesterinsenkern, Antiarrythmika, Gefäßerweiterern, Mitteln gegen Angina, Antihypertensiva, Gefäßschutzmitteln, Antibiotika, Pilzbekämpfungsmitteln, Virenbekämpfungsmitteln, Krebsbekämpfungsmitteln, Entzündungshemmern, Schmerzmitteln, Antiepilektika, Mittel gegen die Parkinson-Krankheit, Neuroleptika, Hypnotika, Anxiolytika, Psychostimulanzien, Mitteln gegen Migräne, Antidepressiva, Hustenmitteln, Antihistaminika oder Antiallergika.

9. Formulierung nach dem vorstehenden Anspruch, **dadurch gekennzeichnet, dass** der Wirkstoff aus den folgenden Verbindungen ausgewählt ist: Metformin, Acetylsalicylsäure, Pentoxifyllin, Prazosin, Acyclovir, Nifedipin, Diltiazem, Naproxen, Ibuprofen, Flurbiprofen, Ketoprofen, Fenoprofen, Indomethacin, Diclofenac, Fentiazac, Oestradiol, Valerat, Metoprolol, Sulpirid, Captopril, Cimetidin, Zidovudin, Nicardipin, Terfenadin, Atenolol, Salbutamol, Carbamazepin, Ranitidin, Enalapril, Simvastatin, Fluoxetin, Alprazolam, Famotidin, Ganciclovir, Famciclovir, Spironolacton, 5-asa Quinidin, Morphin, Pentazocin, Paracetamol, Omeprazol, Metoclopramid und ihre Gemische.

10. Formulierung nach einem der Ansprüche 6 oder 7, **dadurch gekennzeichnet, dass** der Wirkstoff ausgewählt ist aus einer Liste von aktiven Ernährungsverstärkern, Antioxidanzien, Stimulanzien wie etwa Mineralsalze, besonders Eisensalze, Vitamine, Harnstoff, Koffein.

11. Verwendung einer Zusammensetzung nach einem der Ansprüche 1 bis 5 zur Verkapselung eines pharmazeutischen, alimentären oder tierärztlichen Wirkstoffs in einer galenischen Formulierung zur kontrollierten Freisetzung.

**12.** Verfahren zur Erzeugung einer Formulierung nach einem der Ansprüche 6 bis 9, umfassend die folgenden Schritte:

a) Erhitzen einer Zusammensetzung nach einem der Ansprüche 1 bis 5 auf eine Temperatur höher als oder gleich 40 °C, um die Zusammensetzung zum Schmelzen zu bringen;
b) Mischen des pharmazeutischen, alimentären oder tierärztlichen Wirkstoffs in der aus Schritt a) hervorgegangenen geschmolzenen Zusammensetzung;
c) Abkühlen des Gemischs, um die Verkapselung oder den Überzug des Wirkstoffs zu erhalten;

**dadurch gekennzeichnet, dass** die HLB-Werte der Tenside und deren Massenanteile in der im Schritt a) eingeführten Zusammensetzung in Abhängigkeit von der gezielten Dauer der späteren Freisetzung des Wirkstoffs ausgewählt werden.

## Claims

**1.** Composition insoluble in water, with a solid aspect at a temperature of less than or equal to 20°C, comprising the following for 100% of its mass:

- X1% by mass of at least one lipophilic surfactant with an HLB value, H1, greater than or equal to 1 and less than 10;
- X2% by mass of a least one hydrophilic surfactant with an HLB value, H2, greater than or equal to 10 and less than 20;

**characterised by** the fact that its HLB = X1.H1 + X2.H2, X1 and X2 varying from 2 to 60 and **characterised in that** it contains no acrylic polymer and/or acetate succinate, and **characterised in that**:

- said at least one lipophilic surfactant is a sorbitan stearate, and
- said hydrophilic surfactant is chosen among ethoxylated sorbitan esters, ethoxylated alcohols or acids, polyglycerol esters, glucose esters, block copolymers of ethylene and propylene oxides.

**2.** Composition according to the preceding claim, **characterised in that** H1 is greater than or equal to 3.4 and less than or equal to 6 and H2 is greater than or equal to 14 and less than or equal to 16.

**3.** Composition according to one of the preceding claims, **characterised in that** it comprises two to five surfactants each having an HLB, Hi, and a proportion by mass Xi such that the HLB value of said composition is equal to $\sum$ Xi.Hi, where i varies between 2 and 5, xi is between 2% and 60% and $\sum$ Xi $\leq$ 100%.

**4.** Composition according to one of the preceding claims, **characterised in that** said at least one lipophilic surfactant is chosen from among:

- fatty acid and sugar esters; said fatty acids being chosen from among stearic, palmitic, cetostearic, arachidic, behenic acids; said sugars for example being glucose, sorbitol, mannitose, mannitol, sucrose, mannose, xylitol or xylose;
- fatty acid and glycerol esters, said fatty acids being chosen from among stearic, palmitic, cetostearic, arachidic, behenic acids;
- fatty alcohol and sugar ethers, the fatty alcohols being stearic, palmitic, cetostearic, arachidic, behenic alcohols; said sugars for example being reducing sugars, and more particularly glucose, xylose, arabinose, mannose or sucrose;
- divalent salts of fatty acids such as magnesium, zinc or calcium salts of stearic, palmitic, cetostearic, arachidic, behenic acids;
- condensed fatty alcohols with propylene oxide and/or butylene oxide;
- copolymers of block alkoxides (ethylene, propylene, butylene, etc.), rich in propylene or butylene oxide.

**5.** Composition according to one of the preceding claims, **characterised in that** it also comprises up to 100% of its mass, a quantity of additives such as a perfume, an aroma, a palatability enhancer, a colorant, a pigment or a diluent solid at a temperature less than or equal to 20°C such as a hydrogenated vegetable oil such as soy oil, castor oil, rapeseed oil or hydrogenated palm oil, shea butter, a wax such as beeswax or carnauba wax.

**6.** Galenic formulation with controlled release comprising the following for 100% of its mass:

- from 20% to 95% by mass of at least one pharmaceutical, alimentary or veterinary active substance; and
- from 5% to 80% by mass of a composition as defined in one of claims 1 to 5.

**7.** Formulation according the preceding claim, **characterised in that** it comprises the following for 100% of its mass:

- from 50% to 90% by mass of at least one pharmaceutical, alimentary or veterinary active substance; and
- from 10% to 50% by mass of a composition as defined in one of claims 1 to 6.

**8.** Formulation according to one of claims 6 or 7, **characterised in that** the active substance is chosen from among anti-ulcer drugs, antidiabetics, anticoagulants, antithrombotics, lipid-lowering agents, antiarrhythmics, vasodilators, antianginals, antihypertensives, vasoprotectors, antibiotics, antifungals, antivirals, anticancers, antiinflammatory drugs, analgesics, antiepileptics, antiparkinson agents, neuroleptics, hypnotics, anxiolytics, psychostimulants, antimigraine agents, antidepressants, antitussives, antihistamines or antiallergic agents.

**9.** Formulation according the preceding claim, **characterised in that** the active substance is chosen from among the following compounds: metformin, acetylsalicylic acid, pentoxifylline, prazosin, acyclovir, nifedipine, diltiazem, naproxen, ibuprofen, flurbiprofen, ketoprofen, fenoprofen, indomethacin, diclofenac, fentiazac, oestradiol valerate, metoprolol, sulpiride, captopril, cimetidine, zidovudine, nicardipine, terfenadine, atenolol, salbutamol, carbamazepine, ranitidine, enalapril, simvastatin, fluoxetine, alprazolam, famotidine, ganciclovir, famciclovir, spironolactone, 5-asa, quinidine, morphine, pentazocine, paracetamol, omeprazole, metoclopramide and mixtures thereof.

**10.** Formulation according to claim 6 or 7, **characterised in that** the active substance is chosen from among nutritional, fortifying agents, antioxidants, stimulants such as mineral salts and particularly iron salts, vitamins, urea and caffeine.

**11.** Use of a composition as defined in one of claims 1 to 5 for encapsulation of a pharmaceutical, alimentary or veterinary active substance in a galenic formulation with controlled release.

**12.** Method of preparation of a formulation as defined in one of claims 6 to 9 comprising the following steps:

a) heating a composition as defined in one of claims 1 to 5 to a temperature greater than or equal to 40°C in order to melt said composition;
b) mixing said pharmaceutical, alimentary or veterinary active substance in the melted composition obtained in step a),
c) cooling the mix in order to encapsulate or coat said active substance,

**characterised in that** the HLB values of the surfactants and their proportions by mass used in the composition introduced in step a) are chosen as a function of the targeted duration of the subsequent release of said active substance.

Libération d'un sulfate métallique
% de cation métallique libéré
100
90
80
70
60
50
40
30
20
10
0
Temps (heures)
0
1
2
3
4
5
6
7
8
9
Montane 60 100%
Montane 60: 90%

Figure1

% Actif libéré
70.00
60.00
50.00
40.00
30.00
20.00
10.00
0.00
0
10
20
30
40
50
Temps [min.]
Ref. 5
Ref. 4
Ref. 3
Ref. 7
Ref. 6
Ref. 8
Ref. 2
Ref. 1
Ref. 1
Ref. 2
Ref. 3
Ref. 4
Ref. 5
Ref. 6
Ref. 7
Ref. 8


Figure 2

% actif libéré apres 15 min
35
30
25
20
15
10
5
0
0
2
4
6
8
10
HLB

Figure 3

% actif vétérinaire libéré
100
90
80
70
60
50
40
30
20
10
0
0
1
2
3
4
5
6
7
Temps (Heures)
enrobage 12%
enrobage 16%
enrobage 8%
actif non enrobé
profil cible

Figure 4

0.9
0.8
0.7
0.6
0.5
0.4
0.3
0.2
0.1
0
Actif libéré (DO)
0
2
4
6
8
Temps (heures)
Oleate de sorbitan 20OE 0%
Oleate de sorbitan 20OE 5%

Figue 5

80
70
60
50
40
30
20
10
0
% d'actif libéré
0
1
2
3
4
5
6
7
Temps (heures)
stéarate de sorbitan 20OE : 0%
stéarate de sorbitan 20OE : 3%
Cible

Figue 6

Libération de Diclofénac encapsulé
100,0
90,0
80,0
70,0
60,0
50,0
40,0
30,0
20,0
10,0
0,0
% Diclofénac libéré
solution pH 1
solution pH 6.8
0
1
2
3
4
5
6
Temps (en h)

Figure 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2004060074 A1
- US 6835397 B
- CN 101513392
- WO 2007018943 A